# EUROPÄISCHE PATENTSCHRIFT

(11) **EP 4 324 412 B1**
(45) Veröffentlichungstag und Bekanntmachung des Hinweises auf die Patenterteilung: **28.01.2026**
(21) Anmeldenummer: 23185057.9
(22) Anmeldetag: 12.07.2023
(51) Int. Cl.: A61B 17/32, A61M 1/00, A61B 17/16, A61F 2/46, A61B 17/322, A61B 10/02

(54) **AUFBEREITUNGSANORDNUNG**
CONDITIONING ASSEMBLY
DISPOSITIF DE TRAITEMENT

(30) Priorität: 19.08.2022 DE 102022121044
(43) Veröffentlichungstag der Anmeldung: 21.02.2024
(73) Patentinhaber: H & B Electronic GmbH & Co. KG, 75392 Deckenpfronn (DE)
(72) Erfinder: Morlok, Tobias, 71159 Mötzingen (DE); Bayerbach, Jan, 75365 Calw (DE); Heimsch, Jochen, 70734 Fellbach (DE); Remy, Nico, 72070 Tübingen (DE)
(74) Vertreter: Wacker, Jost Oliver

(56) Entgegenhaltungen:
- JP-A- 2006 230 686
- US-A1- 2002 161 449
- US-A1- 2017 258 849
- US-A1- 2019 262 050
- US-A1- 2020 061 258

## Beschreibung

Die Erfindung betrifft eine Aufbereitungsanordnung für ein von einem Patienten entnommenes und nach einer Aufbereitung wieder zu applizierendes Gewebematerial, wie insbesondere ein autologes Knorpelgewebe eines Gelenks oder ein Knochenmaterial, nach dem Oberbegriff des Anspruchs 1 sowie ein Sammel-Aufbereitungssystem mit einer solchen Aufbereitungsanordnung. Die Aufbereitungsanordnung weist dabei einen Sammelbehälter mit einer Sammelaufnahme zur Aufnahme von Gewebematerial auf, das mittels einer Gewebeentnahmevorrichtung beziehungsweise einem Shaver abgetragen wurde. Zudem weist die Aufbereitungsanordnung einen Aufbereitungsbehälter auf, der eine Mischaufnahme bildet, die zur gemeinsamen Aufnahme des Gewebematerials mit einem flüssigen bis pastösen Medium dient, das beispielsweise durch ein Hydrogel gebildet ist oder ein autologes konditioniertes Plasma beziehungsweise Blutserum enthält. Ferner weist die Aufbereitungsanordnung eine Aufbereitungsvorrichtung auf, die zum Vermischen beziehungsweise zur Suspensierung des Gewebematerials im Medium dient, um eine pastöse Applikationsmasse herzustellen.

Unter der Bezeichnung AutoCart ^{™} ist ein System bekannt, das zur Gewinnung von autologem Gewebematerial, zur Herstellung eines Blutserums, zur Herstellung einer pastösen Masse aus dem Gewebematerial und dem Blutserum und zur Applikation der pastösen Masse dient. Dabei wird das von einer Gewebeentnahmevorrichtung abgetragene und zerkleinerte Gewebematerial aus einem Fluidstrom in einen Sammelbehälter abgetrennt, aus dem es dann entnommen wird, um es in eine Spritze zu geben. Diese Spritze wird über einen Adapter mit einer zweiten Spritze verbunden, in der das Blutserum aufgenommen ist. Durch mehrmaliges Vor- und Zurückschieben der beiderseitigen Spritzenkolben werden beide Spritzeninhalte zu der pastösen Applikationsmasse vermischt.

Nachteilig an dem bekannten System ist, dass es beim Umfüllen des Gewebematerials vom Sammelbehälter in die Spritze zu Verunreinigungen beziehungsweise zu einer Keimbelastung kommen kann. Diese Gefahr von Verunreinigungen ist dabei noch größer, falls, je nach Anwendung, eine zusätzliche Zerkleinerung der Partikel des Gewebematerials erforderlich sein sollte. Zudem ist die bekannte Vorgehensweise zur Vermischung des Gewebematerials mit dem Blutserum relativ zeitaufwändig und führt häufig zu einer unzureichenden Homogenität der Applikationsmasse.

US 2020/0061258 A1 offenbart eine Anordnung zur Deagglomeration von abgesaugtem Fett, um dieses, nach der Verarbeitung zu einem klumpenfreien Medium, wieder minimalinvasiv injizieren zu können. Die Anordnung weist hierzu zwei Spritzen auf, die über ein siebartig wirkendes Verbindungelement derart miteinander verbunden sind, dass ein aufgenommenes Fett unter jeweiligem Durchtritt durch das Verbindungselement zwischen beiden Spritzen hin und her befördert werden kann. An den Durchtrittsöffnungen des Verbindungselementes sind dabei Schneidkanten vorgesehen, mittels denen im Fett enthaltene Klumpen zerkleinert werden können.

JP 2006 230686 A beschreibt eine Vorrichtung zur Herstellung von Knochentransplantaten mit einem spritzenförmigen Flüssigkeitsbehälter zur Aufnahme und Abgabe einer flüssigen Komponente, einen Feststoffbehälter zur Aufnahme und Abgabe eines Feststoffes und einen Mischbehälter, in den die flüssige Komponente und der Feststoff injiziert werden können. Der Mischbehälter ist dabei durch einen Rüttler beaufschlagbar, um die flüssige Komponente und den Feststoff zu vermischen.

US 6 071 284 A offenbart eine Aufbereitungsanordnung für ein von einem Patienten entnommenes und nach Aufbereitung zu applizierendes Gewebematerial, wie insbesondere autologes Knorpel- oder Knochengewebe, mit einem Sammelbehälter, mit einem Aufbereitungsbehälter und mit einer Aufbereitungsvorrichtung.

Die Aufgabe der Erfindung ist es, bei einer gattungsgemäßen Aufbereitungsanordnung die genannten Nachteile zu vermeiden und eine sterile Aufbereitung des Gewebematerials sowie eine hohe Homogenität der hergestellten Applikationsmasse zu ermöglichen.

Diese Aufgabe wird durch eine Aufbereitungsanordnung mit den Merkmalen des Anspruchs 1 gelöst. Dabei ist eine Verbindungseinrichtung vorgesehen, an der zur Ausbildung einer kassettenartigen Einschubeinheit durch gemeinsame Aufnahme des Sammelbehälters und des Aufbereitungsbehälters erste Befestigungsmittel zur Anbringung des Sammelbehälters und zweite Befestigungsmittel zur Anbringung des Aufbereitungsbehälters sowie eine in einer Befestigungsstellung zwischen der Sammelaufnahme und der Mischaufnahme positionierte Durchtrittsöffnung vorgesehen sind, wobei die kassettenartige Einschubeinheit zur Durchführung eines Aufbereitungsvorganges in eine Aufbereitungsaufnahme der Aufbereitungsvorrichtung einschiebbar ist. Hierdurch kann der Sammelbehälter über die Verbindungseinrichtung mit dem Aufbereitungsbehälter verbunden werden, um eine direkte Abgabe des Gewebematerials aus dem Sammelbehälter in den Aufbereitungsbehälter zu ermöglichen. Auf diese Weise wird eine freie Entnahme des Gewebematerials aus dem Sammelbehälter heraus vermieden, bei der es zu einer Verunreinigung kommen könnte. Vielmehr können der Sammelbehälter und der Aufbereitungsbehälter mittels der Befestigungsmittel nach außen abgedichtet an der Verbindungseinrichtung befestigt werden, um eine keimfreie Übergabe des Gewebematerials zu ermöglichen.

In einer besonders vorteilhaften Ausführungsform ist an der Durchtrittsöffnung ein Zerkleinerungswerkzeug vorgesehen. Hierdurch kann bei der nach außen abgeschirmten Übergabe des Gewebematerials vom Sammelbehälter in den Aufnahmebehälter zusätzlich eine Zerkleinerung des Gewebematerials auf eine gewünschte Partikelgröße vorgenommen werden. Somit muss das Gewebematerial auch nicht zum Zwecke einer zusätzlichen Zerkleinerung aus dem Sammelbehälter frei entnommen werden.

Bevorzugterweise ist das Zerkleinerungswerkzeug dabei durch eine an der Verbindungseinrichtung verdrehbar gelagerte Scheibe mit wenigstens einem Schneidelement gebildet. Durch das scheibenförmige Zerkleinerungswerkzeug kann für das gesamte übergebene Gewebematerial eine maximale Partikelgröße eingestellt werden. Zudem kann das scheibenförmige Zerkleinerungswerkzeug besonders einfach in die Verbindungseinrichtung integriert werden, wobei es im nicht angetriebenen Zustand die Durchtrittsöffnung verschließt und bei Aktivierung die Übergabe des Gewebematerials an den Aufbereitungsbehälter in Verbindung mit der Zerkleinerung durchführt. Das scheibenförmige Zerkleinerungswerkzeug kann dabei vorteilhafterweise in eine bestimmte Drehstellung verlagert werden, in der die Durchtrittöffnung im wesentliche vollständig abgedichtet ist. Auf diese Weise kann vermieden werden, dass das Gewebematerial vorzeitig und unzerkleinert vom Sammelbehälter in den Aufbereitungsbehälter hinein austritt und/oder dass das Gewebematerial, das Medium oder das hieraus hergestellte Gemisch während des Vermischungsvorganges vom Aufbereitungsbehälter in den Sammelbehälter gelangt.

Zudem ist es günstig, wenn an dem Aufbereitungsbehälter ein von außen antreibbares Mischwerkzeug in die Mischaufnahme ragt. Auf diese Weise kann an dem Aufbereitungsbehälter ein Mischwerkzeug integriert werden, das an die Form und Größe des Aufbereitungsbehälters sowie an die aufzubereitende Menge des Gewebematerials sowie des damit zu vermischenden Mediums angepasst ist. Hierdurch kann eine optimierte Beaufschlagung und Durchmischung des im Aufbereitungsbehälter aufgenommenen Inhalts gewährleistet werden.

Vorteilhafterweise ist das Mischwerkzeug durch einen am Aufbereitungsbehälter verdrehbar gelagerten und von der Aufbereitungsvorrichtung antreibbaren Quirl gebildet, wodurch ein besonders homogenes Durchmischen des Gewebematerials mit dem jeweiligen Medium gewährleistet werden kann.

Zudem weist der Sammelbehälter bevorzugterweise ein Schließelement auf, das zwischen einer Schließstellung, in der es die Sammelaufnahme verschließt, und einer Offenstellung verstellbar ist, in der die Sammelaufnahme wenigstens teilweise geöffnet ist. Hierdurch kann der Sammelbehälter in einem sowohl von der Sammelanordnung als auch von der Aufbereitungsanordnung getrennten Zustand sicher verschlossen werden, um beispielsweise beim Umsetzen von der Sammelanordnung zur Aufbereitungsanordnung eine Verunreinigung des aufgenommenen Gewebematerials zu vermeiden.

Dabei ist es günstig, wenn das Schließelement beim Verbinden der ersten Befestigungsmittel der Verbindungseinrichtung mit ersten Gegenbefestigungsmitteln des Sammelbehälters selbsttätig in eine zweite Offenstellung verbringbar ist, in der eine Behälteröffnung des Sammelbehälters 8 freigegeben ist. Auf diese Weise ist gewährleistet, dass die Sammelaufnahme im verbundenen Zustand des Sammelbehälters mit der Verbindungseinrichtung zur Abgabe des Gewebematerials an den Aufbereitungsbehälter bereitsteht.

Vorteilhafterweise bilden die ersten Befestigungsmittel und die ersten Gegenbefestigungsmittel eine erste Einschubverbindung, wobei das Schließelement durch ein Schiebeelement gebildet ist, das durch eine Einschubbewegung der ersten Befestigungsmittel gegenüber den ersten Gegenbefestigungsmitteln von der Schließstellung in die zweite Offenstellung verbringbar ist. Auf diese Weise kann die Sammelaufnahme auch während der Herstellung der ersten Einschubverbindung des Sammelbehälters mit der Verbindungseinrichtung permanent nach außen hin abgeschirmt werden, um Verunreinigungen zu vermeiden.

Der Aufbereitungsbehälter weist bevorzugterweise ein Verschlusselement auf, das zwischen einer Abschlussstellung, in der es die Mischaufnahme verschließt, und einer Aufnahmestellung verstellbar ist, in der die Mischaufnahme geöffnet ist. Hierdurch kann der Aufbereitungsbehälter auch in einem von der Verbindungseinrichtung beziehungsweise vom Sammelbehälter getrennten Zustand sicher verschlossen werden, um vor einem Aufbereitungsvorgang eine Verunreinigung des darin aufgenommenen Mediums und/oder nach einem Aufbereitungsvorgang eine Verunreinigung der dann aufgenommenen Applikationsmasse zu vermeiden. Zudem ermöglichen das in die zweite Offenstellung verbringbare Schließelement des Sammelbehälters und das in die Aufnahmestellung verstellbare Verschlusselement des Aufbereitungsbehälters, dass das aufgenommene Gewebe beziehungsweise die daraus hergestellte Applikationsmasse in jedem beliebigen Prozessschritt bedarfsweise entnommen werden kann. Auf diese Weise steht das gesammelte Gewebematerial sowohl in unbearbeiteter, in zerkleinerter oder in bereits mit dem Medium vermischter Form ganz oder teilweise auch jederzeit für eine anderweitige Bearbeitung oder Verwendung zur Verfügung.

Dabei ist es günstig, wenn das Verschlusselement beim Verbinden der zweiten Befestigungsmittel der Verbindungseinrichtung mit zweiten Gegenbefestigungsmitteln des Aufbereitungsbehälters selbsttätig in die Aufnahmestellung verbringbar ist. Auf diese Weise ist gewährleistet, dass die Mischaufnahme im verbundenen Zustand des Aufbereitungsbehälters mit der Verbindungseinrichtung zur Aufnahme des Gewebematerials aus dem Sammelbehälter bereitsteht.

Vorteilhafterweise bilden die zweiten Befestigungsmittel und die zweiten Gegenbefestigungsmittel eine zweite Einschubverbindung, wobei das Verschlusselement durch einen Verschlussschieber gebildet ist, der durch eine Einschubbewegung der zweiten Befestigungsmittel gegenüber den zweiten Gegenbefestigungsmitteln durch einen Kontakt mit der Verbindungseinrichtung von der Abschlussstellung in die Aufnahmestellung verbringbar ist. Auf diese Weise kann die Mischaufnahme auch während der Herstellung der zweiten Einschubverbindung des Aufbereitungsbehälters mit der Verbindungseinrichtung permanent nach außen hin abgeschirmt werden, um Verunreinigungen zu vermeiden.

In einer besonders bevorzugten Ausführungsform weist die Aufbereitungsvorrichtung neben der Aufbereitungsaufnahme für die Verbindungseinrichtung mit daran befestigtem Sammelbehälter und Aufbereitungsbehälter wenigstens einen Antrieb auf, wobei der Antrieb in einer Aufnahmestellung eine erste Antriebsverbindung mit dem Mischwerkzeug bildet. Durch die gemeinsame Aufnahme des Sammelbehälters und des Aufbereitungsbehälters im verbundenen Zustand innerhalb der Aufbereitungsvorrichtung kann auch während der Aufbereitung eine permanente Abschirmung der Behälterinhalte und der daraus hergestellten Applikationsmasse sichergestellt werden. Dabei ermöglicht der Antrieb der Aufbereitungsvorrichtung einen maschinellen Mischvorgang.

Vorteilhafterweise weist der wenigstens eine Antrieb in der Aufnahmestellung zusätzlich eine zweite Antriebsverbindung mit dem Zerkleinerungswerkzeug auf. Auf diese Weise kann auch die Zerkleinerung des Gewebematerials bei der Abgabe aus dem Sammelbehälter mittels der Aufbereitungsvorrichtung maschinell vorgenommen werden.

Zudem ist es günstig, wenn der Sammelbehälter einen Kolben zum Entleeren der Sammelaufnahme in Richtung der Durchtrittsöffnung aufweist, um einen vollständigen Auswurf des aufgenommenen Gewebematerials zu ermöglichen.

Bevorzugterweise weist der Aufbereitungsbehälter wenigstens einen verschließbaren Eingabe-/Entnahmeanschluss auf. Über den Eingabe-/Entnahmeanschluss kann dabei die Applikationsmasse beispielsweise in einem spritzenartigen Applikator aufgenommen werden, um nachfolgend aus diesem in eine zu behandelnde Läsion appliziert werden zu können. Ferner kann über den wenigstens einen Eingabe-/Entnahmeanschluss während eines Aufbereitungsvorganges bei Bedarf zusätzliches Medium in die Mischaufnahme eingegeben werden. Dabei ist beispielsweise ein erster Eingabe-/Entnahmeanschluss, der in Form, Größe und Position auf einen bestimmten Applikator abgestimmt ist, sowie ein zweiter Eingabe-/Entnahmeanschluss vorgesehen, der in seiner Form, Größe und Position zum Zweck einer komfortablen Eingabe des Mediums ausgelegt ist.

Vorteilhafterweise ist dabei am Aufbereitungsbehälter ein Schieber zum Entleeren der Mischaufnahme vorgesehen, an dem der wenigstens eine Eingabe-/Entnahmeanschluss gehalten ist, um die Mischaufnahme über diesen zu entleeren. Der Schieber ermöglicht dadurch eine im Wesentlichen vollständige Entnahme der hergestellten pastösen Applikationsmasse und eine leichtere und gleichmäßigere Befüllung des betreffenden Applikators.

Vorteilhafterweise weist die Aufbereitungsvorrichtung zusätzlich wenigstens einen Stellantrieb zur Betätigung des Kolbens und/oder des Schiebers auf, um eine selbsttätige beziehungsweise maschinelle Abgabe des Gewebematerials aus dem Sammelbehälter und/oder eine Ausgabe der aufbereiteten Applikationsmasse aus dem Aufbereitungsbehälter zu ermöglichen. Hierdurch wird nach der Aufnahme der miteinander verbundenen Sammel- und Aufbereitungsbehälter an der Aufbereitungsvorrichtung eine weitestgehend selbsttätige Durchführung des gesamten Aufbereitungsvorganges ermöglicht. Hierbei können die einzelnen Aufbereitungsschritte bestehend aus der Abgabe des Gewebematerials, dem Zerkleinern des Gewebematerials, dem Mischen des Gewebematerials mit dem Medium und der Ausgabe der hergestellten Applikationsmasse wenigstens teilweise selbsttätig von der Aufbereitungsvorrichtung ausgeführt werden. Zusätzlich oder alternativ hierzu kann der Schieber auch manuell betätigt werden, wie beispielsweise mittels eines zu befüllenden Applikators. Auf diese Weise kann die Mischaufnahme auch unabhängig von der Aufbereitungsvorrichtung vollständig entleert werden, insbesondere um die hergestellte Applikationsmasse aus dem Aufbereitungsbehälter heraus möglichst gleichmäßig und vollständig an den Applikator abgeben zu können.

Hierbei weist die Aufbereitungsvorrichtung bevorzugterweise eine Elektronikeinrichtung zur wenigstens teilweise automatisierten Aktivierung des wenigstens einen Antriebs und/oder des wenigstens einen Stellantriebes auf. Hierdurch können alle oder einzelne der Aufbereitungsschritte teil- oder vollautomatisch durchgeführt und zeitlich aufeinander abgestimmt werden.

In einer besonders bevorzugten Ausführungsform ist an der Aufbereitungsvorrichtung zudem eine Kamera vorgesehen, die zur bildlichen Erfassung der Mischaufnahme dient. Hierdurch ist eine permanente optische Überwachung des Aufbereitungsvorganges und der dabei hergestellten Applikationsmasse möglich, wie beispielsweise an einem mit der Kamera verbundenen Bildschirm. Der Bildschirm kann dabei beispielsweise durch einen Touchscreen der Aufbereitungsvorrichtung gebildet sein, der auch zur Eingabe von Steuerungsanweisungen dient. Alternativ oder zusätzlich hierzu kann ferner auch eine automatisierte Auswertung der erfassten Bildinformationen vorgesehen sein, um beispielsweise die Aufbereitungsvorrichtung während eines Aufbereitungsvorganges zu steuern beziehungsweise zu regeln.

Ferner wird die oben genannte Aufgabe durch ein Sammel-/Aufbereitungssystem mit einer Aufbereitungsanordnung in einer der oben genannten Ausführungsformen gelöst, wobei der Sammelbehälter zusätzlich an einem Anschlussgehäuse einer Sammelvorrichtung lösbar festgelegt werden kann, die zum Abtrennen und Sammeln von Gewebe aus einer Fluidströmung zwischen einer Gewebeentnahmevorrichtung und einer Unterdruckvorrichtung angeordnet ist. Auf diese Weise kann das betreffende Gewebematerial nach dem Abtrag über die Gewebeentnahmevorrichtung im Sammelbehälter gesammelt, nach dem Abnehmen des Sammelbehälters von der übrigen Sammelvorrichtung und dessen Verbinden mit dem Aufbereitungsbehälter in diesen abgegeben werden, in der Aufbereitungsvorrichtung aufbereitet und anschließend an den Applikator abgegeben werden, ohne dass es aus einem der Behälter frei entnommen werden muss. Vielmehr ist das Gewebematerial beziehungsweise die daraus hergestellte Applikationsmasse, vom Sammeln bis zur Aufnahme im Applikator durchgängig nach außen hin abgeschirmt und gegen Verunreinigungen geschützt aufgenommen. Auf diese Weise kann eine weitestgehende Sterilität der Applikationsmasse gewährleistet werden.

Vorteilhafterweise bildet der Sammelbehälter einen Strömungsabschnitt eines Verbindungspfades, der in einer Sammelstellung zwischen einem mit der Gewebeentnahmevorrichtung strömungsmäßig verbindbaren shaverseitigen Strömungsanschluss und einem mit der Unterdruckvorrichtung strömungsmäßig verbindbaren unterdruckseitigen Strömungsanschluss herstellbar ist. Hierdurch kann das von der Sammelvorrichtung aus einem Fluidstrom zwischen Gewebeentnahmevorrichtung und Unterdruckvorrichtung abgetrennte Gewebematerial beim Abtrennen direkt im abnehmbaren Sammelbehälter aufgenommen werden. Auf diese Weise steht das Gewebematerial nach dem Abnehmen des Sammelbehälters unmittelbar zur Verfügung für die weitere Aufbereitung.

Bevorzugterweise ist das Schließelement des Sammelbehälters beim Abnehmen des Sammelbehälters vom Anschlussgehäuse selbsttätig von einer ersten Offenstellung, in der die Zutrittsöffnung freigegeben ist, in die Schließstellung verbringbar, um eine durchgehend geschützte Aufnahme des aufgenommenen Gewebematerials sicherstellen zu können. Hierzu kann das Schließelement beispielsweise durch eine Federeinrichtung in die Schließstellung vorgespannt sein.

Es wird darauf hingewiesen, dass alle oben beschriebenen Merkmale des erfindungsgemäßen Gegenstandes untereinander austauschbar beziehungsweise kombinierbar sind, sofern ein Austausch oder eine Kombination derselben aus technischen Gründen nicht ausgeschlossen ist.

In den Figuren ist eine beispielhafte Ausführungsform der Erfindung dargestellt. Es zeigen:
- Figur 1: eine perspektivische Ansicht eines Sammel-/Aufbereitungssystems mit einer erfindungsgemäßen Aufbereitungsanordnung,
- Figur 2: einen Längsschnitt durch eine Sammelvorrichtung der Sammelanordnung nach Figur 1,
- Figur 3: das Sammel-/Aufbereitungssystems nach Figur 1 mit einer für einen Aufbereitungsvorgang vorbereiteten Einschubeinheit,
- Figur 4: eine Ansicht der Einschubeinheit nach Figur 3 in einem getrennten Zustand
- Figur 5: einen Schnitt durch die Einschubeinheit nach Figur 3 im anwendungsbereiten Zustand,
- Figur 6: eine perspektivische Ansicht einer Aufbereitungsvorrichtung der Aufbereitungsanordnung mit aufgenommener Einschubeinheit und
- Figur 7: einen Schnitt durch die Aufbereitungsvorrichtung nach Figur 6.

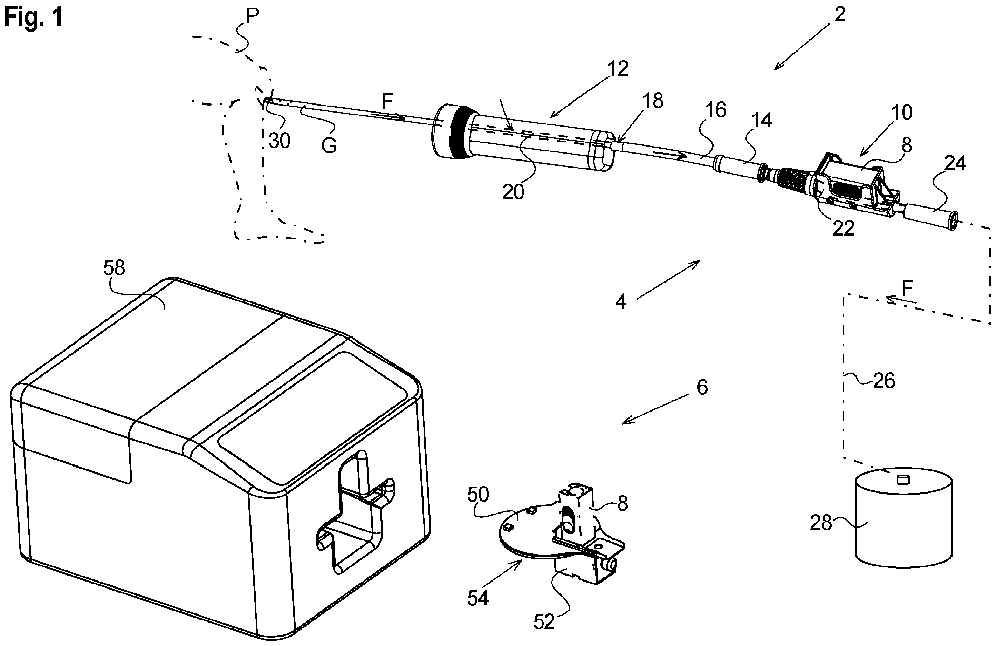

Fig. 1 zeigt ein Sammel-/Aufbereitungssystem 2 für ein von einem Patienten P zu entnehmendes und nach Aufbereitung zu applizierendes Gewebematerial G, wie insbesondere ein autologes Knorpelgewebe eines Gelenks oder ein Knochenmaterial. Dabei bildet das Sammel-/Aufbereitungssystem zum Sammeln des Gewebematerials G eine Sammelanordnung 4 und zum Aufbereiten des gesammelten Gewebematerials G eine hierzu separate Aufbereitungsanordnung 6. Die Sammelanordnung 4 und die Aufbereitungsanordnung 6 werden dabei in Verbindung mit einem gemeinsamen Sammelbehälter 8 verwendet.

Die Sammelanordnung 4 weist eine Sammelvorrichtung 10 für eine durch einen Shaver geformte Gewebeentnahmevorrichtung 12 auf, mittels der das Gewebematerial G, wie insbesondere Knorpel- oder Knochengewebe, vom Patienten P entnommen werden kann. Die Entnahme des Gewebematerials G erfolgt dabei beispielsweise an einem Knie- oder Schultergelenk oder von einem Knochen. Das hierbei gewonnene Gewebematerial G dient zur späteren Verwendung beim Aufbau von Gewebe beziehungsweise Knochen an einem Defekt des Patienten P.

Zum Sammeln des Gewebematerials G im Sammelbehälter 8 weist die Sammelvorrichtung 10 einen shaverseitigen Strömungsanschluss 14 auf, der über eine Verbindungsleitung 16 strömungsmäßig mit einem Abgabeanschluss 18 einer Gewebeabführung 20 der Gewebeentnahmevorrichtung 12 verbunden ist. Zudem weist die Sammelvorrichtung 10 einen mit dem shaverseitigen Strömungsanschluss 14 über einen Verbindungspfad 22 verbindbaren unterdruckseitigen Strömungsanschluss 24 auf, der über eine Anschlussleitung 26 mit einer Unterdruckvorrichtung 28 strömungsmäßig verbunden ist.

Sobald mittels der Unterdruckvorrichtung 28 ein Unterdruck an der Anschlussleitung 26 angelegt wird, wird dadurch ein Fluidstrom F von der Gewebeentnahmevorrichtung 12 über die Sammelvorrichtung 10 zur Unterdruckvorrichtung 28 erzeugt. Mittels der Gewebeabführung 20 kann dabei das von einem Werkzeug 30 abgetragene Gewebematerial G zum Abgabeanschluss 18 transportiert werden, um es dem Sammelbehälter 8 der Sammelvorrichtung 10 zuzuführen.

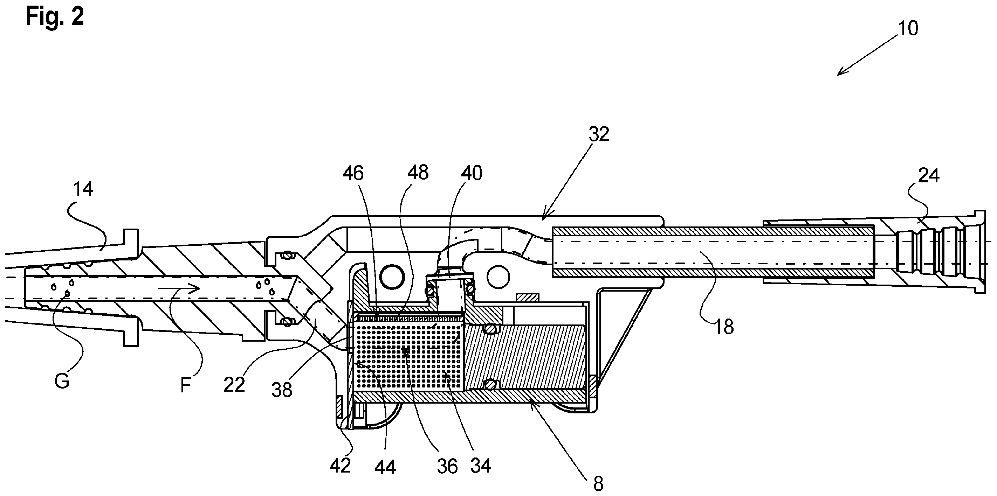

Wie insbesondere aus Figur 2 zu entnehmen ist, ist der Sammelbehälter 8 hierzu derart an einem Anschlussgehäuse 32 festgelegt, dass er mit einer Sammelaufnahme 34 einen Strömungsabschnitt 36 zwischen einer Zutrittsöffnung 38 und einer Austrittsöffnung 40 bildet. Die Zutrittsöffnung 38 ist dabei in ein Schließelement 42 integriert, das in einer dargestellten ersten Offenstellung lediglich die Zutrittsöffnung 38 freigibt und im Übrigen eine Behälteröffnung 44 des Sammelbehälters 8 verschließt. Die Sammelaufnahme 34 ist hierbei teilweise durch eine Siebeinrichtung 46 begrenzt, die in Strömungsrichtung des Fluidstroms F wenigstens eine ebene Siebfläche 48 vor der Austrittsöffnung 40 aufweist.

Wie aus Figur 2 ferner zu entnehmen ist, verläuft der Verbindungspfad 18 vom shaverseitigen Strömungsanschluss 14 über den Strömungsabschnitt 36 zum unterdruckseitigen Strömungsanschluss 24. Auf diese Weise kann das im Fluidstrom F mitgeführte Gewebematerial G mittels der Siebeinrichtung 46 abgetrennt und in der Sammelaufnahme 34 gesammelt werden.

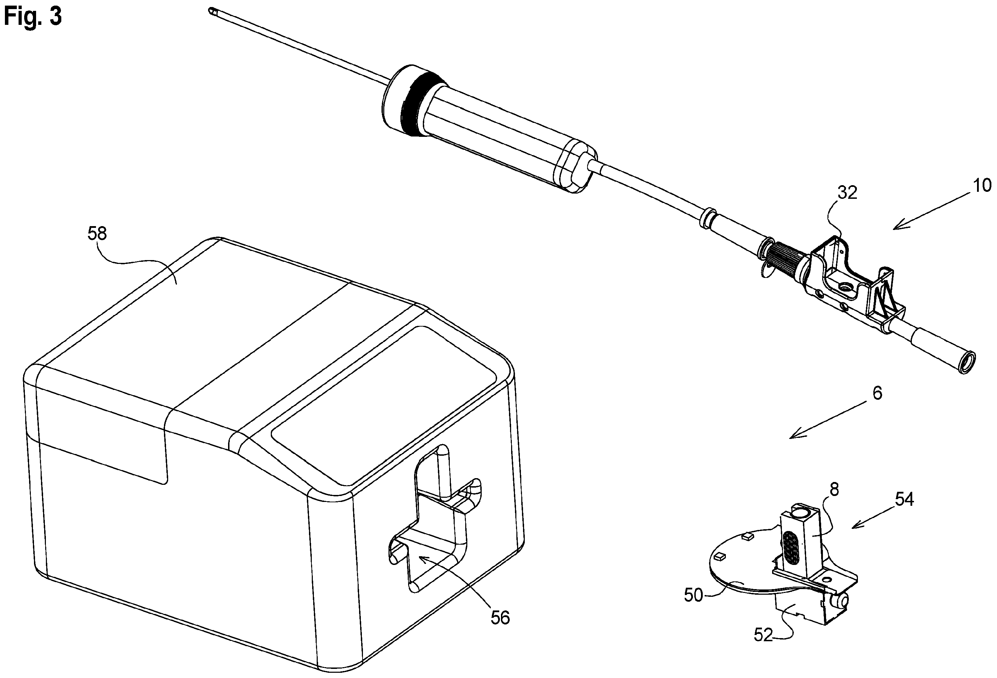

Sobald eine ausreichende Menge des Gewebematerials G gesammelt wurde, kann der Sammelbehälter 8 vom Anschlussgehäuse 32 der Sammelvorrichtung 10 getrennt werden, um gemäß Figur 3 an einer Verbindungseinrichtung 50 der separaten Aufbereitungsanordnung 6 angebracht zu werden. Die Verbindungseinrichtung 50 dient dabei zur gemeinsamen Aufnahme des Sammelbehälters 8 und eines Aufbereitungsbehälters 52, um eine kassettenartige Einschubeinheit 54 auszubilden. Diese kann zur Durchführung eines Aufbereitungsvorganges in eine Aufbereitungsaufnahme 56 einer Aufbereitungsvorrichtung 58 eingeschoben werden.

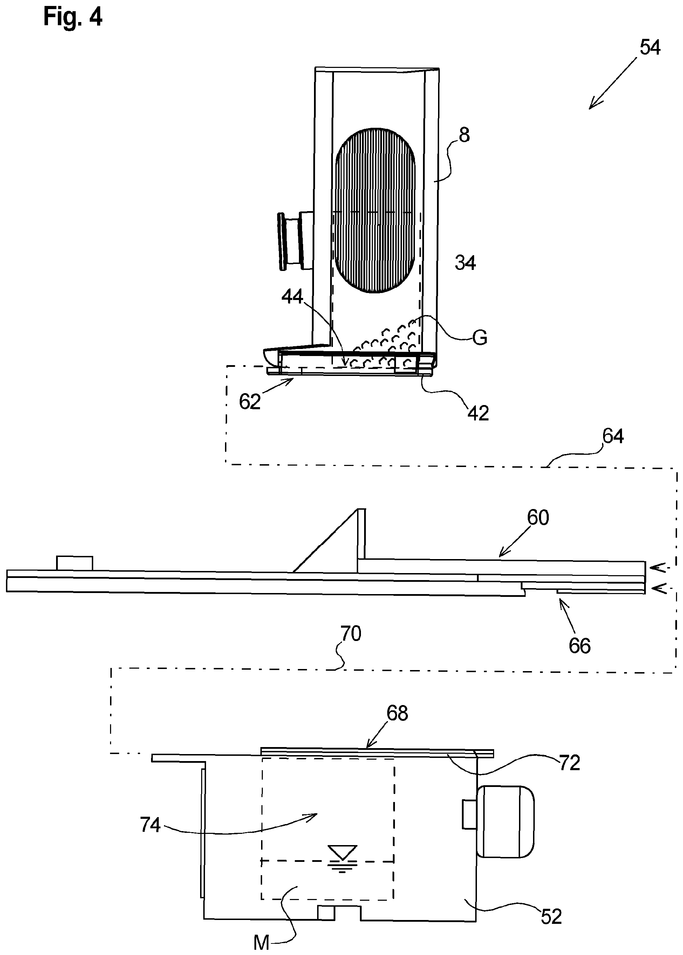

Um beim Umsetzen des Sammelbehälters 8 von der Sammelanordnung 4 zur Aufbereitungsanordnung 6 eine geschützte und nach außen abgeschirmte Aufbewahrung des gesammelten Gewebematerials G zu gewährleisten, wird das Schließelement 42 beim Abnehmen des Sammelbehälters 8 vom Anschlussgehäuse 32 von der ersten Offenstellung gemäß Figur 2 selbsttätig in eine Schließstellung verbracht, in der sowohl die Behälteröffnung 44 als auch die Zutrittsöffnung 38 verschlossen ist, wie in Figur 4 dargestellt. Hierzu kann das Schließelement 42 beispielsweise in Richtung der Schließstellung vorgespannt sein oder es sind Mitnahmemittel vorgesehen, die das Schließelement 42 durch die Relativbewegung gegenüber dem Anschlussgehäuse 32 in die Schließstellung verlagern (nicht dargestellt). Das in der Sammelaufnahme 34 aufgenommene Gewebematerial G kann in jedem Fall nicht mehr über die Behälteröffnung 44 oder die Zutrittsöffnung 38 austreten und ist gegenüber Verunreinigungen von außen geschützt, sobald das Schließelement 42 in der Schließstellung angeordnet ist.

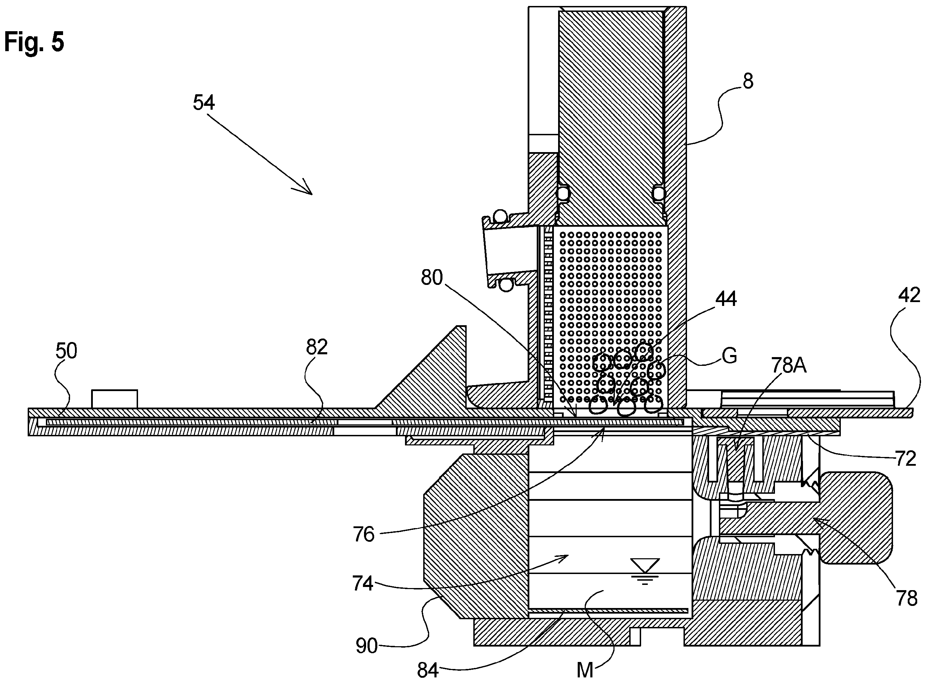

Zur Befestigung des Sammelbehälters 8 an der Verbindungseinrichtung 50 weist diese erste Befestigungsmittel 60 auf, die mit ersten Gegenbefestigungsmitteln 62 des Sammelbehälters 8 zusammenwirken. Die ersten Befestigungsmittel 60 und Gegenbefestigungsmittel 62 weisen dabei beispielsweise ineinandergreifende Schienenelemente auf, die eine erste Einschubverbindung 64 ermöglichen, bei deren Herstellung das schieberförmige Schließelement 42 des Sammelbehälters 8, selbsttätig in eine zweite Offenstellung gemäß Figur 5 verlagerbar ist. In dieser zweiten Offenstellung gibt das Schließelement 42 die Behälteröffnung 44 zumindest weitestgehend frei.

Wie in Figur 4 ferner dargestellt, ist an einer vom Sammelbehälter 8 abgewandten Seite der Verbindungseinrichtung 50 zur Ausbildung der Einschubeinheit 54 gleichzeitig auch der Aufbereitungsbehälter 52 anbringbar.

Zur Befestigung des Aufbereitungsbehälters 52 weist die Verbindungseinrichtung 50 hierbei zweite Befestigungsmittel 66 auf, die mit zweiten Gegenbefestigungsmitteln 68 des Aufbereitungsbehälters 52 zusammenwirken. Die zweiten Befestigungsmittel 66 und Gegenbefestigungsmittel 68 weisen dabei beispielsweise ebenfalls ineinandergreifende Schienenelemente auf, die eine zweite Einschubverbindung 70 ermöglichen, bei deren Herstellung ein Verschlusselement 72 des Aufbereitungsbehälters 52 von einer Abschlussstellung gemäß Figur 4, in der eine Mischaufnahme 74 verschlossen ist, selbsttätig in eine Aufnahmestellung gemäß Figur 5 verlagerbar ist, in der eine Aufnahmeöffnung 76 der Mischaufnahme 74 freigegeben ist. Das Verschlusselement 72 ist hierzu beispielsweise schieberförmig ausgebildet und über einen Kontakt an der Verbindungseinrichtung 50 in Folge der Einschubbewegung verlagerbar.

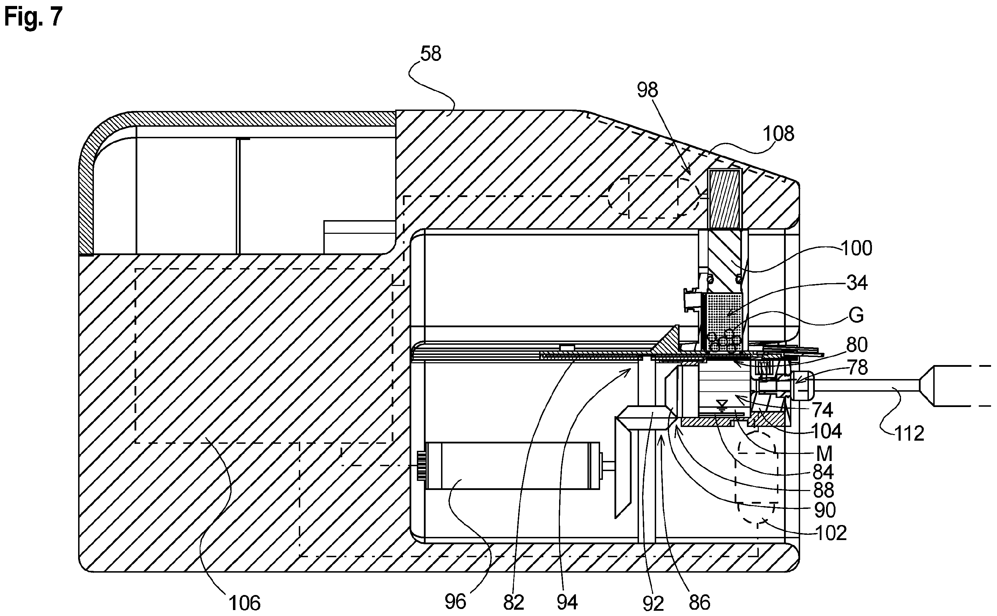

Die Mischaufnahme 74 des Aufbereitungsbehälters 52 dient zur gemeinsamen Aufnahme und Aufbereitung des Gewebematerials G mit einem flüssigen bis pastösen Medium M, das beispielsweise durch ein Hydrogel oder ein Blutserum gebildet ist. Das Medium M kann hierzu, wie in Figur 5 dargestellt, bereits vorportioniert in der Mischaufnahme 74 aufgenommen sein und/oder während eines Aufbereitungsvorganges über wenigstens einen verschließbaren Eingabe-/Entnahmeanschluss 78 oder 78A des Aufbereitungsbehälters 52 in die Mischaufnahme 74 eingegeben werden. Dabei ist beispielsweise ein erster Eingabe-/Entnahmeanschluss 78, der in Form, Größe und Position auf einen bestimmten Applikator 112 (siehe Figur 7) abgestimmt ist, sowie ein zweiter Eingabe-/Entnahmeanschluss 78A vorgesehen, der in seiner Form, Größe und Position zum Zweck einer komfortablen Eingabe des Mediums M ausgelegt ist.

Wie aus Figur 5 ferner zu entnehmen ist, weist die Verbindungseinrichtung 50 eine Durchtrittsöffnung 80 auf, die in der dargestellten Befestigungsstellung der Einschubeinheit 54 mit angebrachtem Sammelbehälter 8 und Aufbereitungsbehälter 52 zwischen der Behälteröffnung 44 und der Aufnahmeöffnung 76 angeordnet ist. Zudem weist die Verbindungseinrichtung 50 ein Zerkleinerungswerkzeug 82 auf, das sich über die Durchtrittsöffnung 80 erstreckt. Das Zerkleinerungswerkzeug 82 ist beispielsweise scheibenförmig ausgebildet und verdrehbar in der Verbindungseinrichtung 50 gehalten. Dabei weist das Zerkleinerungswerkzeug 82 wenigstens ein Schneidelement vorzugsweise eine Vielzahl von Scheidelementen auf. Das scheibenförmige Zerkleinerungswerkzeug 82 kann dabei vorteilhafterweise in eine bestimmte Drehstellung verlagert werden, in der die Durchtrittöffnung 80 im wesentliche vollständig abgedichtet ist. Auf diese Weise kann vermieden werden, dass das Gewebematerial G vorzeitig und unzerkleinert vom Sammelbehälter 8 in den Aufbereitungsbehälter 52 hinein austritt und/oder dass das Gewebematerial G, das Medium M oder das hieraus hergestellte Gemisch während des Vermischungsvorganges vom Aufbereitungsbehälter 52 in den Sammelbehälter 8 gelangt.

Zudem ist an dem Aufbereitungsbehälter 52 ein Mischwerkzeug 84 vorgesehen, das beispielsweise durch einen verdrehbar gelagerten Quirl gebildet ist, der in die Mischaufnahme 74 ragt.

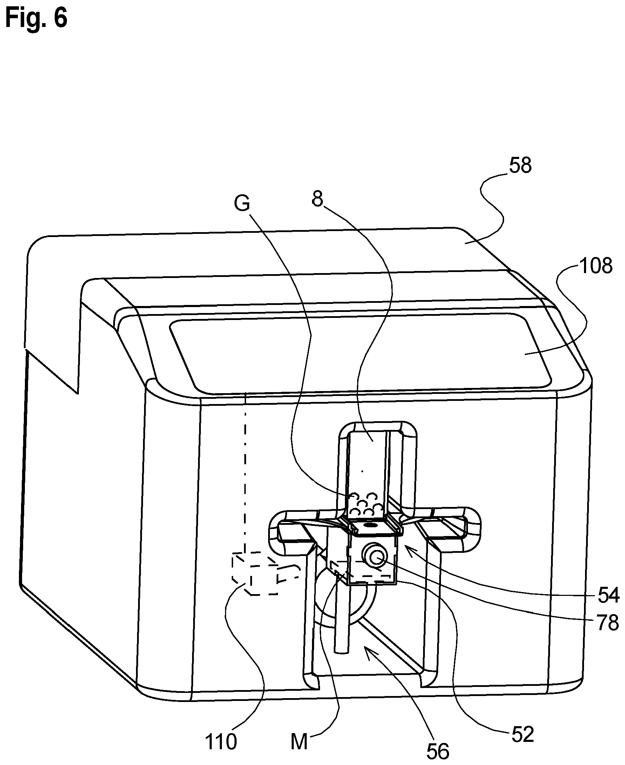

Wie aus Figur 6 zu entnehmen ist, kann die Einschubeinheit 54 in der Art einer Kassette in die Aufbereitungsaufnahme 56 der Aufbereitungsvorrichtung 58 geschoben werden. In dieser Aufnahmestellung kann das im Sammelbehälter 8 aufgenommene Gewebematerial G und das im Aufbereitungsbehälter 52 aufgenommene Medium M durch Beaufschlagung mittels der Aufbereitungsvorrichtung 58 zu der gewünschten Applikationsmasse verarbeitet werden.

Wie aus Figur 7 zu entnehmen ist, weist die Aufbereitungsvorrichtung 58 hierzu einen ersten Antrieb 86 auf, der in der Aufnahmestellung eine erste Antriebsverbindung 88 mit Kupplungsmitteln 90 des Mischwerkzeuges 84 herstellt. Zudem weist die Aufbereitungsvorrichtung 58 einen zweiten Antrieb 92 auf, der in der Aufnahmestellung eine zweite Antriebsverbindung 94 mit dem Zerkleinerungswerkzeug 82 herstellt. Der erste Antrieb 86 und der zweite Antrieb 92 können dabei, wie dargestellt, durch Teile eines gemeinsamen Getriebes gebildet sein, das wiederum von einem gemeinsamen Motor 96 angetrieben werden kann. Alternativ hierzu können beide Antriebe 86, 92 auch separat ausgebildet und aktivierbar sein (nicht dargestellt).

Ferner weist die Aufbereitungsvorrichtung 58 einen ersten Stellantrieb 98 auf, mittels dem ein Kolben 100 des Sammelbehälters 8 in Richtung der Behälteröffnung 44 verschoben werden kann, um die Sammelaufnahme 34 zu entleeren. Zudem weist die Aufbereitungsvorrichtung 58 einen zweiten Stellantrieb 102 auf, mittels dem ein Schieber 104 des Aufbereitungsbehälters 52 verschoben werden kann, an dem der Eingab-/Entnahmeanschlusses 78 gehalten ist, um die Mischaufnahme 74 über diesen zu entleeren. Zusätzlich oder alternativ hierzu kann der Schieber 104 auch manuell betätigt werden, wie beispielsweise mittels des mit dem Eingab-/Entnahmeanschlusses 78 verbundenen Applikators 112. Auf diese Weise kann die Mischaufnahme 74 auch unabhängig von der Aufbereitungsvorrichtung 58 vollständig entleert werden, beispielsweise um eine hergestellte Applikationsmasse aus dem Aufbereitungsbehälter 52 heraus an den Applikator 112 abzugeben.

Ferner weist die Aufbereitungsvorrichtung 58 eine Elektronik 106 auf, mittels der die Antriebe 86, 92 beziehungsweise deren Motor 96 sowie die Stellantriebe 98, 102 aktivierbar sind. Dabei kann vorgesehen sein, dass die Elektronik 106 einen Aufbereitungsvorgang wenigstens teilautomatisiert, vorteilhafterweise vollautomatisiert, ablaufen lässt. Eine Bedienung beziehungsweise Programmierung der Elektronik 106 ist dabei beispielsweise über einen Touchscreen 108 möglich. An diesem kann zudem eine Ausgabe von Bildinformation erfolgen, die mittels einer Kamera 110 generiert werden, die auf die Mischaufnahme 74 gerichtet ist, wie in Figur 6 dargestellt.

Während eines Aufbereitungsvorganges wird in der Aufnahmestellung gemäß Figur 7 durch Aktivierung des Motors 96 über den ersten Antrieb 86 das quirlförmige Mischwerkzeug 84 in Bewegung gesetzt und gleichzeitig über den zweiten Antrieb 92 das scheibenförmige Zerkleinerungswerkzeug 82 in Drehbewegung versetzt. Hierdurch wird das in der Sammelaufnahme 34 gehaltene Gewebematerial G zerkleinert und gelangt über die Durchtrittsöffnung 80 in den Aufbereitungsbehälter 52, wo es mit dem Medium M vermischt werden kann.

Durch Aktivierung des ersten Stellantriebes 98 kann dabei der Kolben 100 verschoben werden, um eine vollständige Zerkleinerung und eine vollständige Abgabe des Gewebematerials G an die Mischaufnahme 74 sicherzustellen.

Nach der Abgabe des zerkleinerten Gewebematerials G an die Mischaufnahme 74 wird dieses solange mit dem Medium M vermischt, bis eine geeignete Applikationsmasse hergestellt ist. Bei Bedarf kann hierzu, insbesondere über den Eingabe-/Entnahmeanschluss 78A, zusätzliches Medium M in die Mischaufnahme 74 eingegeben werden.

Nach Fertigstellung der Applikationsmasse kann der Eingabe-/Entnahmeanschluss 78 mit dem Applikator 112 in Form einer Spritze verbunden werden, der mit der Applikationsmasse aufgezogen wird. Um das Aufziehen des Applikators 112 zu unterstützen und die Mischaufnahme 74 möglichst vollständig entleeren zu können, kann dabei der zweite Stellantrieb 102 aktiviert werden, um den Schieber 104 mit dem Eingabe-/Entnahmeanschluss 78 zu verlagern.

Es wird darauf hingewiesen, dass alle oben beschriebenen Elemente und Merkmale der verschiedenen Ausführungsformen des erfindungsgemäßen Gegenstandes untereinander austauschbar beziehungsweise kombinierbar sind, sofern ein Austausch oder eine Kombination derselben aus technischen Gründen nicht ausgeschlossen ist.

## Patentansprüche

1. Aufbereitungsanordnung (6) für ein von einem Patienten (P) entnommenes und nach Aufbereitung zu applizierendes Gewebematerial (G), wie insbesondere autologes Knorpel- oder Knochengewebe,
mit einem Sammelbehälter (8), der eine Sammelaufnahme (34) zur Aufnahme des mittels einer Gewebeentnahmevorrichtung (12) entnommenen Gewebematerials (G) aufweist,
mit einem Aufbereitungsbehälter (52) der eine Mischaufnahme (74) zur gemeinsamen Aufnahme des Gewebematerials (G) und eines flüssigen bis pastösen Mediums (M) aufweist,
und mit einer Aufbereitungsvorrichtung (58) zur Vermischung des Gewebematerials (G) und des Mediums (M),
**dadurch gekennzeichnet, dass** eine Verbindungseinrichtung (50) vorgesehen ist, an der zur Ausbildung einer kassettenartigen Einschubeinheit (54) durch gemeinsame Aufnahme des Sammelbehälters (8) und des Aufbereitungsbehälters (52) erste Befestigungsmittel (60) zur Anbringung des Sammelbehälters (8) und zweite Befestigungsmittel (66) zur Anbringung des Aufbereitungsbehälters (52) sowie eine zwischen der Sammelaufnahme (34) und der Mischaufnahme (74) positionierbare Durchtrittsöffnung (80) vorgesehen sind, wobei die kassettenartige Einschubeinheit (54) zur Durchführung eines Aufbereitungsvorganges in eine Aufbereitungsaufnahme (56) der Aufbereitungsvorrichtung (58) einschiebbar ist.

2. Aufbereitungsanordnung nach Anspruch 1, **dadurch gekennzeichnet, dass** an der Durchtrittsöffnung (80) ein Zerkleinerungswerkzeug (82) vorgesehen ist, wobei das Zerkleinerungswerkzeug (82) insbesondere durch eine an der Verbindungseinrichtung (50) verdrehbar gelagerte Scheibe mit wenigstens einem Schneidelement gebildet ist.

3. Aufbereitungsanordnung nach Anspruch 1 oder 2, **dadurch gekennzeichnet, dass** an dem Aufbereitungsbehälter (52) ein Mischwerkzeug (84) in die Mischaufnahme (74) ragt, wobei das Mischwerkzeug (84) insbesondere durch einen am Aufbereitungsbehälter (52) verdrehbar gelagerten und von der Aufbereitungsvorrichtung (58) antreibbaren Quirl gebildet ist.

4. Aufbereitungsanordnung nach einem der Ansprüche 1 bis 3, **dadurch gekennzeichnet, dass** der Sammelbehälter (8) ein Schließelement (42) aufweist, das zwischen einer Schließstellung, in der es die Sammelaufnahme (34) verschließt, und einer Offenstellung verstellbar ist, in der die Sammelaufnahme (34) wenigstens teilweise geöffnet ist.

5. Aufbereitungsanordnung nach Anspruch 4, **dadurch gekennzeichnet, dass** das Schließelement (42) beim Verbinden der ersten Befestigungsmittel (60) der Verbindungseinrichtung (50) mit ersten Gegenbefestigungsmitteln (62) des Sammelbehälters (8) selbsttätig in eine zweite Offenstellung verbringbar ist, in der eine Behälteröffnung (44) des Sammelbehälters (8) geöffnet ist.

6. Aufbereitungsanordnung nach Anspruch 5, **dadurch gekennzeichnet, dass** die ersten Befestigungsmittel (60) und die ersten Gegenbefestigungsmittel (62) eine erste Einschubverbindung (64) bilden und das Schließelement (42) schieberförmig ausgebildet und durch eine Einschubbewegung der ersten Befestigungsmittel (60) gegenüber den ersten Gegenbefestigungsmitteln (62) von der Schließstellung in die zweite Offenstellung verbringbar ist.

7. Aufbereitungsanordnung nach einem der Ansprüche 1 bis 6, **dadurch gekennzeichnet, dass** der Aufbereitungsbehälter (52) ein Verschlusselement (72) aufweist, das zwischen einer Abschlussstellung, in der es die Mischaufnahme (74) verschließt, und einer Aufnahmestellung verstellbar ist, in der die Mischaufnahme (74) geöffnet ist, wobei bevorzugterweise das Verschlusselement (72) beim Verbinden der zweiten Befestigungsmittel (66) der Verbindungseinrichtung (50) mit zweiten Gegenbefestigungsmitteln (68) des Aufbereitungsbehälters (52) selbsttätig in die Aufnahmestellung verbringbar ist.

8. Aufbereitungsanordnung nach Anspruch 7, **dadurch gekennzeichnet, dass** die zweiten Befestigungsmittel (66) und die zweiten Gegenbefestigungsmittel (68) eine zweite Einschubverbindung (70) bilden und das Verschlusselement (72) durch einen Verschlussschieber gebildet ist, der durch eine Einschubbewegung der zweiten Befestigungsmittel (66) gegenüber den zweiten Gegenbefestigungsmitteln (68) von der Abschlussstellung in die Aufnahmestellung verbringbar ist.

9. Aufbereitungsanordnung nach einem der Ansprüche 1 bis 8, **dadurch gekennzeichnet, dass** die Aufbereitungsvorrichtung (58) neben der Aufbereitungsaufnahme (56) für die Verbindungseinrichtung (50) mit daran befestigtem Sammelbehälter (8) und Aufbereitungsbehälter (52) wenigstens einen Antrieb (86; 92) die aufweist und der Antrieb (86; 92) in einer Aufnahmestellung eine erste Antriebsverbindung (88) mit dem Mischwerkzeug (84) bildet, wobei bevorzugterweise der wenigstens eine Antrieb (86; 92) in der Aufnahmestellung zusätzlich eine zweite Antriebsverbindung (94) mit dem Zerkleinerungswerkzeug (82) bildet.

10. Aufbereitungsanordnung nach einem der Ansprüche 1 bis 9, **dadurch gekennzeichnet, dass** der Sammelbehälter (8) einen Kolben (100) zum Entleeren der Sammelaufnahme (34) in Richtung der Durchtrittsöffnung (80) aufweist.

11. Aufbereitungsanordnung nach einem der Ansprüche 1 bis 10, **dadurch gekennzeichnet, dass** der Aufbereitungsbehälter (52) einen verschließbaren Eingabe-/Entnahmeanschluss (78) aufweist.

12. Aufbereitungsanordnung nach Anspruch 11, **dadurch gekennzeichnet, dass** am Aufbereitungsbehälter (52) ein Schieber (104) zum Entleeren der Mischaufnahme (74) über den Eingabe-/Entnahmeanschluss (78) vorgesehen ist.

13. Aufbereitungsanordnung nach einem der Ansprüche 10 bis 12, **dadurch gekennzeichnet, dass** die Aufbereitungsvorrichtung (58) wenigstens einen Stellantrieb (98; 102) zur Betätigung des Kolbens (100) und/oder des Schiebers (104) aufweist, wobei die Aufbereitungsvorrichtung (58) insbesondere eine Elektronikeinrichtung (106) zur wenigstens teilweise automatisierten Aktivierung des wenigstens einen Antriebs (86; 92) und/oder des wenigstens einen Stellantriebes (98; 102) aufweist.

14. Aufbereitungsanordnung nach einem der Ansprüche 1 bis 13, **dadurch gekennzeichnet, dass** an der Aufbereitungsvorrichtung (58) eine Kamera (110) zur bildlichen Erfassung der Mischaufnahme (74) vorgesehen ist.

15. Sammel-/Aufbereitungssystem mit einer Aufbereitungsanordnung nach einem der Ansprüche 1 bis 14, **dadurch gekennzeichnet, dass** der Sammelbehälter (8) an einem Anschlussgehäuse (32) einer Sammelvorrichtung (10) lösbar festlegbar ist, die zum Abtrennen und Sammeln von Gewebematerial (G) aus einer Fluidströmung (F) zwischen einer Gewebeentnahmevorrichtung (12) und einer Unterdruckvorrichtung (28) angeordnet ist.

16. Sammel-/Aufbereitungssystem nach Anspruch 15, **dadurch gekennzeichnet, dass** der Sammelbehälter (8) einen Strömungsabschnitt (36) eines Verbindungspfades (22) zwischen einem mit der Gewebeentnahmevorrichtung (12) strömungsmäßig verbindbaren shaverseitigen Strömungsanschluss (14) und einem mit der Unterdruckvorrichtung (28) strömungsmäßig verbindbaren unterdruckseitigen Strömungsanschluss (24) bildet.

17. Sammel-/Aufbereitungssystem nach Anspruch 15 oder 16, **dadurch gekennzeichnet, dass** das Schließelement (42) des Sammelbehälters (8) beim Abnehmen des Sammelbehälters (8) vom Anschlussgehäuse (32) selbsttätig von einer ersten Offenstellung in die Schließstellung verbringbar ist.

## Claims

1. A processing arrangement (6) for tissue material (G) removed from a patient (P) and to be applied after processing, such as in particular autologous cartilage or bone tissue,
comprising: a collection container (8) which has a collection receptacle (34) for receiving the tissue material (G) removed by a tissue removal device (12),
a processing container (52) which has a mixing receptacle (74) for jointly receiving the tissue material (G) and a liquid to pasty medium (M),
and a processing device (58) for mixing the tissue material (G) and the medium (M),
**characterized in that** a connection device (50) is provided on which, for forming a cassette-like slide-in unit (54) by jointly receiving the collection container (8) and the processing container (52), first fasteners (60) for attaching the collection container (8) and second fasteners (66) for attaching the processing container (52) as well as a passage opening (80) which can be positioned between the collection receptacle (34) and the mixing receptacle (74) are provided, wherein the cassette-like slide-in unit (54) can be slid into a processing receptacle (56) of the processing device (58) for carrying out a processing operation.

2. Processing arrangement according to claim 1, **characterized in that** a comminuting tool (82) is provided at the passage opening (80), wherein the comminuting tool (82) is formed in particular by a disc rotatably mounted on the connection device (50) and has at least one cutting element.

3. Processing arrangement according to claim 1 or 2, **characterized in that** a mixing tool (84) projects into the mixing receptacle (74) on the processing container (52), wherein the mixing tool (84) is formed in particular by an agitator rotatably mounted on the processing container (52) and drivable by the processing device (58).

4. Processing arrangement according to any one of claims 1 to 3, **characterized in that** the collection container (8) has a closing element (42) which can be adjusted between a closed position in which it closes the collection receptacle (34) and an open position in which the collection receptacle (34) is at least partially open.

5. Processing arrangement according to claim 4, **characterized in that** the closing element (42) can be brought automatically into a second open position in which a container opening (44) of the collection container (8) is open when the first fasteners (60) of the connection device (50) are connected to first counter-fasteners (62) of the collection container (8).

6. Processing arrangement according to claim 5, **characterized in that** the first fasteners (60) and the first counter-fasteners (62) form a first slide-in connection (64) and the closing element (42) is designed in the shape of a slide and can be displaced from the closed position into the second open position by a sliding-in movement of the first fasteners (60) relative to the first counter-fasteners (62).

7. Processing arrangement according to any one of claims 1 to 6, **characterized in that** the processing container (52) has a closing element (72) which can be adjusted between a final position in which it closes the mixing receptacle (74) and a receiving position in which the mixing receptacle (74) is open, wherein preferably the closing element (72) can be brought automatically into the receiving position when the second fasteners (66) of the connection device (50) are connected to second counter-fasteners (68) of the processing container (52).

8. Processing arrangement according to claim 7, **characterized in that** the second fasteners (66) and the second counter-fasteners (68) form a second slide-in connection (70) and the closing element (72) is formed by a closure slide which can be displaced by a slide-in movement of the second fasteners (66) relative to the second counter-fasteners (68) from the final position into the receiving position.

9. Processing arrangement according to any one of claims 1 to 8, **characterized in that** the processing device (58) has, in addition to the processing receptacle (56) for the connection device (50) with collection container (8) and processing container (52) attached thereto, at least one drive (86; 92) and the drive (86; 92) in a receiving position forms a first drive connection (88) with the mixing tool (84), wherein preferably the at least one drive (86; 92) in the receiving position also forms a second drive connection (94) with the comminuting tool (82).

10. Processing arrangement according to any one of claims 1 to 9, **characterized in that** the collection container (8) has a piston (100) for emptying the collection receptacle (34) in the direction of the passage opening (80).

11. Processing arrangement according to any one of claims 1 to 10, **characterized in that** the processing container (52) has a closable input/output connection (78).

12. Processing arrangement according to claim 11, **characterized in that** a slide (104) for emptying the mixing receptacle (74) via the input/output connection (78) is provided on the processing container (52).

13. Processing arrangement according to any one of claims 10 to 12, **characterized in that** the processing device (58) has at least one actuator (98; 102) for actuating the piston (100) and/or the slide (104), wherein the processing device (58) in particular has an electronic device (106) for at least partially automated activation of the at least one drive (86; 92) and/or the at least one actuator (98; 102).

14. Processing arrangement according to any one of claims 1 to 13, **characterized in that** a camera (110) is provided on the processing device (58) for the visual recording of the mixing receptacle (74).

15. Collection/processing system comprising a processing arrangement according to any one of claims 1 to 14, **characterized in that** the collection container (8) can be detachably fixed to a connection housing (32) of a collection device (10) which is arranged for separating and collecting tissue material (G) from a fluid flow (F) between a tissue removal device (12) and a vacuum device (28).

16. Collection/processing system according to claim 15, **characterized in that** the collection container (8) forms a flow portion (36) of a connection path (22) between a shaver-side flow connection (14) which can be fluidically connected to the tissue removal device (12) and a vacuum-side flow connection (24) which can be fluidically connected to the vacuum device (28).

17. Collection/processing system according to claim 15 or 16, **characterized in that** the closing element (42) of the collection container (8) can be brought automatically from a first open position into the closed position when the collection container (8) is removed from the connection housing (32).

## Revendications

1. Agencement de préparation (6) pour un matériau de tissu (G), comme en particulier du tissu cartilagineux ou osseux autologue, prélevé sur un patient (P) et à appliquer après préparation,
avec un récipient collecteur (8) qui présente un logement collecteur (34) pour loger le matériau de tissu (G) prélevé au moyen d'un dispositif de prélèvement de tissu (12),
avec un récipient de préparation (52) qui présente un logement de mélange (74) pour loger conjointement le matériau de tissu (G) et un milieu liquide à pâteux (M),
et avec un agencement de préparation (58) pour mélanger le matériau de tissu (G) et le milieu (M),
**caractérisé en ce qu'**un appareil de connexion (50) est prévu, sur lequel sont prévus de premiers moyens de fixation (60) pour fixer le récipient collecteur (8) et de seconds moyens de fixation (66) pour fixer le récipient de préparation (52) ainsi qu'une ouverture de passage (80) positionnable entre le logement collecteur (34) et le logement de mélange (74) pour former une unité d'insertion de type cassette (54) en logeant conjointement le récipient collecteur (8) et le récipient de préparation (52), dans lequel l'unité d'insertion de type cassette (54) peut être insérée dans un logement de préparation (56) du dispositif de préparation (58) pour mettre en œuvre un processus de préparation.

2. Agencement de préparation selon la revendication 1, **caractérisé en ce qu'**un outil de broyage (82) est prévu au niveau de l'ouverture de passage (80), dans lequel l'outil de broyage (82) est en particulier formé par un disque monté de manière rotative sur l'appareil de connexion (50) et avec au moins un élément de coupe.

3. Agencement de préparation selon la revendication 1 ou 2, **caractérisé en ce qu'**un outil de mélange (84) fait saillie dans le logement de mélange (74) sur le récipient de préparation (52), dans lequel l'outil de mélange (84) est en particulier formé par un agitateur monté de manière rotative sur le récipient de préparation (52) et pouvant être entraîné par l'agencement de préparation (58).

4. Agencement de préparation selon l'une quelconque des revendications 1 à 3, **caractérisé en ce que** le récipient collecteur (8) présente un élément de fermeture (42) qui peut être déplacé entre une position de fermeture, dans laquelle il ferme le logement collecteur (34), et une position d'ouverture, dans laquelle le logement collecteur (34) est au moins partiellement ouvert.

5. Agencement de préparation selon la revendication 4, **caractérisé en ce que** l'élément de fermeture (42) peut être déplacé automatiquement dans une seconde position ouverte, dans laquelle une ouverture (44) du récipient collecteur (8) est ouverte, lors de la connexion des premiers moyens de fixation (60) de l'appareil de connexion (50) avec de premiers moyens de fixation complémentaires (62) du récipient collecteur (8).

6. Agencement de préparation selon la revendication 5, **caractérisé en ce que** les premiers moyens de fixation (60) et les premiers moyens de fixation complémentaires (62) forment une première connexion par insertion (64) et l'élément de fermeture (42) est conçu sous forme de tiroir et peut être amené de la position de fermeture à la seconde position d'ouverture par un mouvement d'insertion des premiers moyens de fixation (60) par rapport aux premiers moyens de fixation complémentaires (62).

7. Agencement de préparation selon l'une quelconque des revendications 1 à 6, **caractérisé en ce que** le récipient de préparation (52) présente un élément de fermeture (72) qui est réglable entre une position de fermeture, dans laquelle il ferme le logement de mélange (74), et une position de logement dans laquelle le logement de mélange (74) est ouvert, dans lequel l'élément de fermeture (72) peut de préférence être déplacé automatiquement dans la position de logement lors de la connexion du second moyen de fixation (66) de l'appareil de connexion (50) avec le second moyen de fixation complémentaire (68) du récipient de préparation (52).

8. Agencement de préparation selon la revendication 7, **caractérisé en ce que** les seconds moyens de fixation (66) et les seconds moyens de fixation complémentaires (68) forment une seconde connexion d'insertion (70) et l'élément de fermeture (72) est formé par un curseur de fermeture qui peut être amené de la position de fermeture à la position de logement par un mouvement d'insertion des seconds moyens de fixation (66) par rapport aux seconds moyens de fixation complémentaires (68).

9. Agencement de préparation selon l'une quelconque des revendications 1 à 8, **caractérisé en ce que** l'agencement de préparation (58) présente, outre le logement de préparation (56) pour l'appareil de connexion (50) avec le récipient collecteur (8) et le récipient de préparation (52) qui y sont fixés, au moins un entraînement (86 ; 92) et l'entraînement (86 ; 92) forme, dans une position de logement, une première connexion d'entraînement (88) avec l'outil de mélange (84), dans lequel l'au moins un entraînement (86 ; 92) forme de préférence, dans la position de logement, une seconde connexion d'entraînement (94) supplémentaire avec l'outil de broyage (82).

10. Agencement de préparation selon l'une quelconque des revendications 1 à 9, **caractérisé en ce que** le récipient collecteur (8) présente un piston (100) pour vider le logement collecteur (34) en direction de l'ouverture de passage (80).

11. Agencement de préparation selon l'une quelconque des revendications 1 à 10, **caractérisé en ce que** le récipient de préparation (52) présente un raccord d'entrée/prélèvement (78) pouvant être fermé.

12. Agencement de préparation selon la revendication 11, **caractérisé en ce qu'**un tiroir (104) est prévu sur le récipient de préparation (52) pour vider le logement de mélange (74) par l'intermédiaire du raccord d'entrée/prélèvement (78).

13. Agencement de préparation selon l'une quelconque des revendications 10 à 12, **caractérisé en ce que** le dispositif de préparation (58) présente au moins un actionneur (98 ; 102) pour actionner le piston (100) et/ou le tiroir (104), dans lequel le dispositif de préparation (58) présente en particulier un appareil électronique (106) pour l'activation au moins partiellement automatisée d'au moins un entraînement (86 ; 92) et/ou de l'au moins un actionneur (98 ; 102).

14. Agencement de préparation selon l'une quelconque des revendications 1 à 13, **caractérisé en ce qu'**une caméra (110) est prévue sur le dispositif de préparation (58) pour la saisie visuelle du logement de mélange (74).

15. Système de collecte/préparation avec un agencement de préparation selon l'une quelconque des revendications 1 à 14, **caractérisé en ce que** le récipient collecteur (8) peut être fixé de manière amovible sur un boîtier de raccordement (32) d'un dispositif collecteur (10) qui est disposé entre un dispositif de prélèvement de tissu (12) et un dispositif à dépression (28) pour séparer et collecter du matériau de tissu (G) à partir d'un écoulement de fluide (F).

16. Système de collecte/préparation selon la revendication 15, **caractérisé en ce que** le récipient collecteur (8) forme une section d'écoulement (36) d'un chemin de connexion (22) entre un raccord d'écoulement côté rasoir (14) pouvant être connecté au dispositif de prélèvement de tissu (12) et un raccord d'écoulement côté dépression (24) pouvant être connecté au dispositif à dépression (28).

17. Système de collecte/préparation selon la revendication 15 ou 16, **caractérisé en ce que** l'élément de fermeture (42) du récipient collecteur (8) peut être déplacé automatiquement d'une première position ouverte à la position fermée lors du retrait du récipient collecteur (8) du boîtier de raccordement (32).
